# EUROPEAN PATENT APPLICATION

(11) **EP 3 591 034 A1**
(43) Date of publication of application: **08.01.2020**
(21) Application number: 18761555.4
(22) Date of filing: 02.02.2018
(51) Int. Cl.: C12N 1/21, C12P 13/20

(54) **METHOD FOR PRODUCING L-ASPARAGINIC ACID**

(30) Priority: 28.02.2017 EA 201700163
(71) Applicant: Akcionernoe Obschestvo "Bioamid", g. Saratov 410033 (RU)
(72) Inventor: SINOLITSKII, Maksim Konstantinovich, Saratov 410054 (RU); SINOLITSKAIA, Stella Vladimirovna, Saratov 410054 (RU); VORONIN, Sergey Petrovich, Saratov 410028 (RU); DEBABOV, Vladimir Georgievich, Moscow 115409 (RU); NOVIKOV, Andrey Dmitrievich, Krasnodar 350049 (RU); YANENKO, Alexandr Stepanovich, Moscow 121293 (RU)
(74) Representative: Rimini, Rebecca
(86) International application number: PCT/RU2018/050006
(87) International publication number: WO 2018/160103

(57) **Abstract**

The group of inventions relates to biotechnology and refers to a method for producing L-aspartic acid. Recombinant *Escherichia coli* strain, an aspartase producer, was designed for this purpose by introducing a recombinant plasmid containing the aspA gene under the control of lactose promoter lacUV5 into *E. coli* VKPM 7188 strain. Producing L-aspartic acid involves biotransformation of ammonium fumarate using a biocatalyst, produced on the basis of the recombinant E. coli strain cells, and the purification of the target product by known methods. Free or immobilized cells of the *E. coli* strain are used as a biocatalyst. The recombinant *E.coli* strain allows to produce a high level of aspartase activity without using isopropyl thiogalactoside as an inducer in the composition of the nutrient medium.

SEQUENCE LISTING

<110> AKTSIONERNOE OBSCHESTVO "BIOAMID"

<120> METHOD FOR PRODUCING L-ASPARAGINIC ACID

<130> 000000300

<150> EA201700163
<151> 2017-02-28

<160> 2

<210> 1
<211> 22
<212> DNA
<213> Artificial Sequence

<400> 1
tttgtataag aaaatgagag gg                                                22

<210> 2
<211> 42
<212> DNA
<213> Artificial Sequence

<400> 2
aaaggatcct gtacgattac tgttcgcttt catcagtata gc                          42

## Description

### Field of the invention

The invention relates to the biotechnology and refers to a method for producing L-aspartic acid, which finds application in the pharmaceutical, food, microbiological industry and in feed production.

### Background of the invention

There are known methods for producing L-aspartic acid by biocatalytic transformation of ammonium fumarate into L-asparaginate ammonium using microorganisms that synthesize the enzyme aspartate ammonia-lyase (aspartase). The use of immobilized bacterial cells is the most promising and technically feasible in the process of biotransformation of ammonium fumarate into L-asparaginate. The immobilized cells fill the flow reactor, and the ammonium fumarate solution is pumped through biocatalyst layer by a pump, and a solution of aspartic acid monoammonium salt is formed at the reactor outlet.

Natural (wild), mutant, and recombinant strains producing aspartase are used, relating to various genera, for example, the genus *Escherichia* (SU659611). Natural strain is used in this invention. The main disadvantage of this method is the low activity of aspartase. Mutant strains are used in the invention according to patent EP0110422. The main disadvantages of this method are the high level of side activity of fumarate hydratase and the low activity of aspartase. A recombinant strain was produced from the genus *Serratia* (EP0111293). The main disadvantages of this method are the lack of a selection factor in the cultivation of the strain and the rapid loss of the plasmid. The genera *Pseudomonas, Bacillus, Brevibacterium,* et al. (EP0752476), the main disadvantages of the method are the complexity, multiple stages and low target activity.

There is a known method for producing L-aspartic acid using a natural strain of *Escherichia coli* (VKPM B-7188) (RU2174558). However, this method is not effective enough due to the low aspartase activity of the strain.

There is a known method for producing aspartic acid, described in patent US4692409. It describes a recombinant *Escherichia coli* TA-5004 strain, with high aspartase activity and a biocatalyst based on the biomass of cells of this strain included in the polysaccharide gel, kappa-carrageenan. The main disadvantages of this method are the lack of a selection factor during cultivation of the strain, rapid plasmid loss, as well as diffusion limitation of the carrageenan gel matrix, which, with an increase in specific aspartase cell activity, does not allow to significantly increase the specific activity of the biocatalyst.

There is also a known method for producing aspartic acid using immobilized cells having aspartase activity on the surface of ion-exchange resin particles or inorganic substances using polyazetidine (US4436813). There is a known method for producing aspartic acid, which uses a biocatalyst based on cells with aspartase activity immobilized on vermiculite particles using crosslinking agents, including glutaraldehyde in the presence of polyethyleneimine (US4504582). Disadvantage of this method is the low efficiency of the resulting biocatalyst.

Closest to the claimed method is a method for producing L-aspartic acid, described in patent US6214589. A biocatalyst based on cells of the recombinant *Escherichia coli* strain was used in this method. The strain contains plasmid pUC19 with the insertion of the aspartase gene under the lactose promoter. The method involves passing an ammonium fumarate solution through a column filled with biocatalyst particles, followed by purification of L-aspartic acid from the reaction solution. The biocatalyst is produced by immobilizing bacterial cells on an ion-exchange resin using polymers that change solubility depending on the pH of the medium.

Disadvantage of this method is the high cost of the medium components, in particular the need to add an expensive inducer of the lactose operon, isopropyl-1-thio-β-D-galactoside (IPTG), which induces aspartase expression. This compound is widely used to induce the expression of recombinant genes in expression vectors designed based on the lac-operon promoter. The need to add this expensive substance reduces the economic efficiency of the process *of E. coli* cell cultivation, biocatalyst production and, as a consequence, the entire L-aspartic acid production process. In addition, the disadvantage of this method is the inaccessibility of polymers and the use of complex equipment for immobilization.

### Summary of the invention

The objective of the present invention is to increase the efficiency of the L-aspartic acid production process by reducing the complexity and reducing the cost of the process associated with the use of a more effective biocatalyst.

The objective is achieved by:
- constructing a recombinant *Escherichia coli* strain, an aspartase producer, by introducing a recombinant plasmid containing the aspA gene under the control of lactose promoter lacUV5 into *E*. *coli* VKPM 7188 strain (Russian National Collection of Industrial Microorganisms);
- in some embodiments of the invention, the objective is achieved by producing a recombinant strain deposited in the Russian National Collection of Industrial Microorganisms with the number *E. coli* VKPM B-11745;
- development of a method for producing L-aspartic acid by biotransformation of ammonium fumarate using a biocatalyst based on cells of a recombinant *E. coli* strain;
- in some embodiments of the invention, the objective is achieved by developing a method for producing L-aspartic acid using free or immobilized *E*. *coli* cells as a biocatalyst;
- in some particular embodiments of the invention, the objective is achieved by developing a method for immobilizing cells of an *E*. *coli* strain in a polyethyleneimine matrix covalently cross-linked with glutaraldehyde.
- in some particular embodiments of the invention, the objective is achieved by developing a method for immobilizing cells of an *E. coli* strain characterized in that the weight ratio of polyethyleneimine and glutaraldehyde ranges from 1:1 to 3:1.
- in some particular embodiments of the invention, the objective is achieved by developing a method for immobilizing cells of an *E. coli* strain characterized in that the weight ratio of polyethyleneimine and glutaraldehyde components to *E. coli* biomass ranges from 0.25:1 to 3:1.

As a result of implementation of the invention, the following technical results are achieved:
- recombinant plasmid pAsp 116-5 is produced to create highly productive *E*. *coli* strains;
- recombinant *E*. *coli* strains are produced, including *E. coli* VKPM B-11745 strain, with high aspartase activity;
- complexity reduction, simplifying and cheapening the process of L-aspartic acid production;
- an increase in the aspartase activity of the biocatalyst based on the recombinant *E. coli* strain;
- an increase of the biocatalyst enzymatic activity without introducing an expensive inducer (IPTG, isopropylthiogalactoside) into the medium;
- elimination of the need to use expensive nutrient media, large amounts of cultivation and inaccessible components for immobilization of cells;
- a method of immobilization of recombinant *E. coli* strain cells has been developed;
- a method for producing L-aspartic acid using a biocatalyst based on free and immobilized *E*. *coli* cells has been developed;
- a method of immobilization of *E*. *coli* in a polyethyleneimine matrix covalently cross-linked with glutaraldehyde has been developed;
- a method of continuous biotransformation of ammonium fumarate by immobilized cells has been developed.

### Description of the drawings

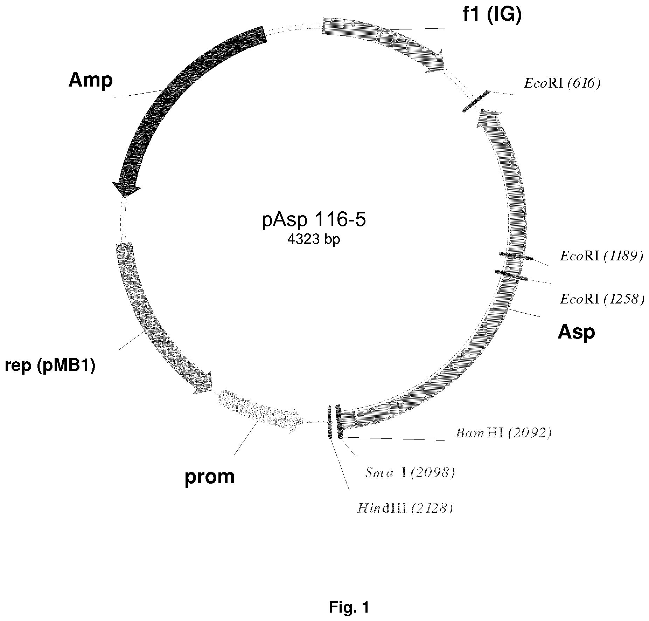

Fig 1. Diagram of plasmid pAsp 116-5.

On the picture: Amp - ampicillin resistance gene, rep (pMB1) - replicon, f1 (IG)- prophage f1 DNA fragment, Asp - L-aspartate ammonia-lyase gene, prom - promoter region LacUV5.

### Detailed description of the invention

The present invention is aimed at improving the efficiency of the L-aspartic acid production process by reducing the complexity and cheapening the process associated with the use of a more effective biocatalyst based on a new recombinant *E*. *coli* strain immobilized with polyethyleneimine cross-linked with glutardialdehyde.

The claimed method consists of providing a high aspartase activity of a biocatalyst based on cells of a new recombinant *E. coli* strain without using an expensive IPTG inducer in the process of its cultivation and producing a highly efficient biocatalyst based on cells of an *E.coli* strain.

The proposed method of producing L-aspartic acid by biotransformation of ammonium fumarate to ammonium asparaginate using a biocatalyst based on microbial *E. coli* cells, followed by the target product purification, involves the use of highly active biocatalyst based on a new recombinant *E.coli* strain grown on a nutrient medium without the addition of the IPTG inducer in the biotransformation process.

During the implementation of the invention, the strain can be used both in a free form and immobilized using techniques known in the art, for example, in a polyacrylamide gel, in carrageenan and in other methods, in particular, the strain can be immobilized in a covalently cross-linked glutaraldehyde polyethyleneimine matrix.

Polyethyleneimine and glutaraldehyde are available and well studied compounds that are widely used for immobilization of enzymes (see, for example, R. Bahulekar et.al., Polyethyleneimine in immobilization of biocatalysts // Enzyme and Microb. Technol., 1991. - V.13(11) - P.858-868.; US4504582). Schiff bases formed in the process of joint cross-linking of the amino groups of bacterial cells and polyethyleneimine are considered not very strong compounds. However, under the alkaline conditions of aspartase functioning, as well as in aqueous solutions with a salt concentration above 20%, this type of compounds is quite stable. In addition, formed polymer matrix has sufficiently wide pores so as not to exert significant diffusion restrictions with a particle size of 0.5 to 1.0 mm.

The method is carried out as follows.

The design of the recombinant strain is performed.

In a variant thereof, a recombinant strain is understood to be a line of microorganisms derived from *Escherichia coli* VKPM B-11745, retaining the main mutations and genetic engineering modifications that ensure high production of the enzyme aspartase. Recombinant *E*. *coli* VKPM B-11745 strain is designed by transforming *E. coli* VKPM B-7188 strain with plasmid pAsp 116-5. The choice of *E*.*coli*VKPM B-7188 strain as the recipient when designing the claimed strain is related to the fact that, despite the low level of aspartase production, it meets most of the requirements imposed on the recipients for expression, i.e. genetically labeled, allows to produce high efficiency during transformation and does not produce toxic metabolites.

The *Escherichia coli* VKPM B-7188 strain is produced from the wild strain by treatment with a mutagen (nitrosoguanidine), followed by selection of mutants on the analog of glucose alpha methyl-D-glucopyranoside (RU2174558).

Plasmid pAsp 116-5 is produced on the basis of vector pUC19 and contains the L-aspartate ammonia-lyase gene from *E*. *coli* B-7188 under lactose promoter lacUV5 and the penicillin antibiotic resistance gene. A diagram of the plasmid is shown in Fig. 1, where: Amp - ampicillin resistance gene, rep (pMB1) - replicon, f1 (IG) - prophage f1 DNA fragment, Asp - L-aspartate ammonia-lyase gene, prom - promoter region LacUV5.

*E.coli* VKPM B-11745 strain is characterized by all the features common to *E.coli,* including, among others, cultural and morphological features. The culture is represented by gram-negative, facultative anaerobic rod-shaped cells, with slightly rounded ends, 0.4-0.8 x 1-3 µm in size. Cells grow well on simple rich nutrient media. Colonies on meat-peptone agar (MPA) and Luria-Bertani medium (LB) are whitish shiny, 2-3 mm in size, the edges are smooth, they do not form spores.

After the cell transformation of *E*. *coli* VKPM B-7188strain - DNA pAsp 116-5, a spontaneous mutant was produced in the process of transformant strain purification, which maintains a high level of aspartase synthesis in the absence of IPTG inducer. Table 1 shows the effect of adding an IPTG inducer in the amount of 1 mM to the fermentation medium.

**Table 1. The influence of IPTG inducer on aspartase activity.**

| Indicator | Without IPTG | IPTG 1 mM |
|---|---|---|
| Specific aspartase activity U/units.OD | 2,570±120 | 2,470±140 |
| Total aspartase activity U/ ml CCL (cell culture fluid) | 53,860±5200 | 47,920±4300 |

The produced *E. coli* 7188 strain (pAsp116-5) is deposited in the Russian National Collection of Industrial Microorganisms and has a registration number VKPM B-11745. This strain, when grown on a fermentation medium in a fermenter, is capable of producing L-aspartate ammonia-lyase in an amount of at least 40,000 U/ml.

The specific aspartase activity of the cells was determined as follows. 0.5 ml of a suspension of pre-activated microbial cells with an optical density of 5 units was added to 4.5 ml of a 1.5 M solution of ammonium fumarate and the mixture was incubated with stirring for one hour at 30°C. The amount of aspartic acid formed was determined by high performance liquid chromatography (HPLC). The formation of one micromole of aspartic acid per hour is taken for a unit of activity (U). The optical density of the cell suspension was determined at a wavelength of 540 nm and an optical path of 5 mm.

The production of immobilized cells was carried out by mixing the cell suspension with a solution of polyethyleneimine homopolymer (Polymin-P, BASF), after which a solution of glutaraldehyde was added, stirred and left to complete the cross-link processes. The amount of polyethyleneimine homopolymer solution was chosen such that the final concentration of polyethyleneimine ranged from 10 to 50% per 100% substance. The amount of glutaraldehyde solution was chosen such that the final concentration of glutaraldehyde ranged from 5 to 30% per 100% substance. The amount of E. coli cell biomass was chosen such that the final concentration of cell biomass ranged from 25 to 65% per dry weight. The ratio of polyethyleneimine and glutaraldehyde ranges from 1:1 to 3:1. And the weight ratio of the components of polyethyleneimine and glutaraldehyde to the *E. coli* biomass ranges from 0.25:1 to 3:1. Where polyethyleneimine and glutaraldehyde is per 100% substance, and biomass is per dry weight.

Then, the produced mass was crushed, dried to a residual moisture content of 65 to 75%, fractions less than 0.5 mm and more than 1.5 mm were sieved out, kept in a solution of ammonium acetate with a concentration of 200 to 250 g/l for at least 24 hours.

The method of immobilization provides producing immobilized cells with specific aspartase activity from 90,000 to 210,000 U/h per gram of wet biocatalyst. This biocatalyst placed in a flow reactor provides, at 25 °C, biotransformation of a solution of ammonium fumarate with a concentration of 200 g/l (fumaric acid) at a rate of 3 to 5 volumes/hour of biocatalyst contained in the reactor with a 99% efficiency.

Production and cultivation of *Escherichia coli* VKPM B-11745 strain, production of immobilized *cells of E. coli* VKPM B-11745 strain, biotransformation of ammonium fumarate and production of L-aspartic acid using immobilized cells of *E*.*coli* VKPM B-11745 strain confirmed by the following embodiment examples.

It should be understood that these and all the examples given in the application materials are not limiting and are given only to illustrate the present invention.

### Example 1. Producing plasmid pAsp 116-5.

The sequence of the AspA gene from *Escherichia coli,* which is the L-aspartate ammonia-lyase gene without the regulatory promoter region, was used to produce plasmid pAsp 116-5.

The aspA sequence was produced by PCR method using genomic DNA purified from *E*. *coli* VKPM B-7188 strain as a template.

The genomic DNA was purified using the Genomic DNA Purification Kit as recommended by the manufacturer (Genomic DNA Purification Kit, # K0512, Thermo Fisher Scientific Biosciences Inc.).

PCR product was produced using the following pair of primers:
ttt gta taa gaa aat gag agg g (SEQ ID NO: 1) and
aaa gga tcc tgt acg att act gtt cgc ttt cat cag tat agc (SEQ ID NO: 2)

DNA fragments were produced using Pfu polymerases.

Primers-oligonucleotides synthesized by the CUC (Common Use Center) of the FSUE (Federal State Unitary Enterprise) GosNllgenetika and the enzymes by Thermo Fisher Scientific Biosciences Inc. were used.

Approximately 100 nmol of each primer was used for the amplification reaction. The fragment of amplified DNA after electrophoresis in 1% agarose gel was purified by extraction using a kit for DNA purification from a gel as recommended by the manufacturer (GeneJET Gel Extraction Kit, # K0691, Thermo Fisher Scientific Biosciences Inc.).

The purified DNA fragment in the amount of 0.5 µg was ligated with 0.2 µg of plasmid pUC19 DNA split with Smal restriction enzyme. Ligation was performed using Thermo Fisher Scientific Biosciences Inc. reagents as recommended by the manufacturer. The produced plasmid was transformed into *E. coli* XL1-Blue by an electroporation method according to the technique described in the source (Sambrook et al., Molecular Cloning, second edition Cold Spring Harbor Laboratory, Plainview, N.Y., 1989). Clones containing the desired DNA insert were selected on plates with LB agar medium with ampicillin and by the standard test for the absence of β-galactosidase activity, as described in the above source. Plasmid DNA purified from selected clones was tested by PCR method for the presence of the insert, using the above primers. Confirmed insert clones were tested for aspartase activity. The nucleotide sequence of the gene in the clones with confirmed aspartase activity was tested by the method of sequencing.

Plasmid DNA purified from selected clones, of confirmed size, confirmed nucleotide sequence, ensuring the expression of the L-aspartate ammonia-lyase in *E. coli* XL1*-*Blue strain was hereinafter referred to as pAsp 116-5.

### Example 2. Producing E.coli VKPM B-11745 strain.

*Escherichia coli VKPM* B-7188 strain for transformation was used to prepare an electro-competent culture as described in the source (Sambrook et al., Molecular Cloning, second edition Cold Spring Harbor Laboratory, Plainview, N.Y., 1989).

Plasmid DNA pAsp 116-5 produced in accordance with the description in Example 1 was used as DNA for transformation of *Escherichia coli* VKPM B-7188 strain.

Selection of transformants was performed on LB agar medium with ampicillin. Selected transformants were tested for the presence of aspartase activity. The phenotype of the transformants was maintained by sieving on the same medium.

Ten clones that showed the greatest aspartase activity were tested for the presence of L-aspartate ammonia-lyase activity in the acetate fermentation medium, as described further in Example 3.

According to the results of cultivation, the most active clone was selected. The selected clone with the highest aspartase activity, which is the transformant of *E*. *coli* VKPM B-7188 (pAsp 116-5) and which allows to produce the activity of L-aspartate ammonia-lyase not less than 10,000 U/ml when cultivated in test tubes, hereinafter referred to as *E. coli* VKPM B-11745.

### Example 3. Cultivation of E.coli VKPM B-11745 strain in flasks.

The strain was pre-grown at 37 °C overnight on LB agar medium with 100 mg/l ampicillin.

Fresh culture was subcultured from the plates by an inoculation loop into test tubes with 10 ml of liquid LB medium with ampicillin and cultivated on a thermostatically controlled shaker at 37 °C, 300 rpm for at least 15 hours.

The produced seed material was subcultured into test tubes with 10 ml of liquid LB medium with ampicillin, diluting 100 times (100 µl of culture per 10 ml of medium). It was cultivated on a thermostatically controlled shaker at 37 °C, 300 rpm, for at least 2 hours until the OD₆₀₀ ∼ 0.6 - 0.8. The produced seed material was subcultured into flasks with 100 ml of liquid fermentation medium of the following composition (g/l):
Na₂HPO₄·12H₂O - 15.0; KH₂POa-2.0; CH₃COONa - 7; Yeast extract - 20; MgSO₄·7H₂O - 0.25; pH - 6.5-7.0 with ampicillin -100 mg/l and cultivated on a thermostatically controlled shaker at 37 °C, 300 rpm, for at least 2 hours to an OD₆₀₀ ∼ 0.6 - 0.8. After that, the cultivation temperature was lowered to 30 °C and cultivation was performed for at least another 7 hours until the maximum culture activity was reached. When cultivating *E.coli* VKPM B-11745 strain, aspartase production was at least 10,000 U/ml.

### Example 4. Cultivation of E.coli VKPM B-11745 strain in a laboratory fermentor.

The strain was pre-grown at 37 °C overnight on LB agar medium with 100 mg/l ampicillin.

Fresh culture was subcultured from the plates by an inoculation loop into two flasks with 50 ml of liquid LB medium with ampicillin and cultivated on a thermostatically controlled shaker at 37 °C, 300 rpm for at least 15 hours.

The produced seed material was transferred into a 3-liter fermenter comprising 2 l of fermentation nutrient media of the following composition (g/l):
KH₂PO₄ - 2.0; CH₃COONa - 13.8; Yeast extract - 50; MgSO₄·7H₂O - 0.25; pH - 6.8-7.2, ampicillin - 100 mg/l. Cultivation conditions: temperature 30°C, aeration - 2 l/min, stirring - 600 rpm, pH 7.8. The automatic pH maintenance system carries out the dosage of the acetic acid solution. Cultivation time 15-18 hours. The optical density of the cell culture fluid at the discharge 30.0 units OD. Specific aspartase activity - 2,400 U/units OD. Total aspartase activity - 72,000 U/ml. The cells were separated by centrifugation and washed with saline. The number of collected wet cells in the form of a paste was 171 g.

### Example 5. Producing immobilized cells.

The biomass of *E*. *coli* B-11745 strain cells from example 4 in an amount of 10 g was placed in a glass beaker, into which 11 g of a 1% solution of sodium chloride was preliminary added and stirred. Then, continuing the stirring, 5 g of a 20% polyethyleneimine homopolymer solution (Polymin-P, BASF), previously neutralized with hydrochloric acid to pH 7.5, was added and further, while stirring, 3 g of a 25% glutaraldehyde solution (Panreac) was added. The mixture began to harden quickly. It was left for 30 minutes to complete the reactions, and then crushed and dried to a residual moisture content of 65-75%. The produced immobilized cells were sieved through a sieve, leaving a fraction of 0.5-1.5 mm, which was placed in a 20% solution of ammonium acetate and left for at least 24 hours at 5 °C.

The produced biocatalyst had a specific activity of 180,900 U/g of a wet biocatalyst.

### Example 6. Continuous biotransformation of ammonium fumarate by immobilized cells.

The biocatalyst produced according to Example 5, in the amount of 5 g (wet weight) was filled into a jacketed glass column (1x12.7 cm, 10 ml). A coolant with a temperature of 25°C was feed into the jacket. Ammonium fumarate solution with a concentration of 200 g/l (fumaric acid), pH 8.5 and with the addition of 1 mM MgSO₄, was pumped through a layer of the biocatalyst by a peristaltic pump. The feed rate of the solution is 50 ml/hour. The solution leaving the column contained less than 1.5 g/l of ammonium fumarate (per fumaric acid), which corresponds to a conversion of more than 99%. The conversion degree remained at more than 99% in the continuation of the continuous process of ammonium fumarate biotransformation for three months or more.

### Example 7. Producing L-aspartic acid from the reaction solutions after biotransformation.

1,600 ml of the reaction solution were added to a 2 liter flask with reflux after the biotransformation according to Example 6. The flask was placed on a magnetic stirrer with heating and the solution was heated with stirring to 90-98 °C. Without stopping the stirring, 150 g of crystalline fumaric acid was introduced into the flask. Heating and maintaining the temperature within 90-95 °C continued for another 30 minutes. Then the heat was turned off and the suspension of precipitated aspartic acid crystals was continued to be stirred. The solution was cooled to 25 °C, kept at this temperature for 30 minutes, and the crystals were separated by filtration. The crystals were washed on the filter with desalted water and dried at 80°C. 269.8 g of crystalline L-aspartic acid were produced. Specific rotation angle in 5 n HCI: [α]D20=+24.9. The admixture of fumaric acid 0.34% The mother solution and the washings were evaporated under vacuum to a volume of 1.3 liters. 90 g of fumaric acid, 25% aqueous ammonia up to a solution pH of 8.5 and demineralized water up to a volume of 1.6 liters were added to the produced concentrate. The produced solution was again sent for biotransformation.

The proposed method provides a more efficient production of L-aspartic acid by reducing the cost and simplifying the process. In addition to the above it eliminates the need to use expensive nutrient media, large amounts of cultivation and inaccessible components for immobilization of cells.

Thus, the proposed method for producing L-aspartic acid allows to ensure high aspartase activity of the biocatalyst, significantly reduce the complexity and cost of the process.

Despite the fact that the invention has been described with reference to the forms of invention embodiment, it should be obvious to the persons skilled in the art that the specific, detailed described experiments are shown for the purpose of illustrating this invention only, and should not be considered as those that in any way confine the scope of the invention. It should be understood that the embodiment of various modifications are possible without deviation from the main point of this invention.

## Claims

1. Recombinant *Escherichia coli* strain, an aspartase producer, produced by introducing a recombinant plasmid containing the aspA gene under the control of lactose promoter lacUV5 into *E. coli* VKPM 7188 strain.

2. The strain according to claim 1, deposited in the Russian National Collection of Industrial Microorganisms with the number VKPM B-11745.

3. The method for producing L-aspartic acid by biotransformation of ammonium fumarate using a biocatalyst based on *E. coli* cells, **characterized in that** the *E*. *coli* strain according to any one of claims 1 or 2 is used as the biocatalyst.

4. The method according to claim 3, **characterized in that** free or immobilized cells of the *E. coli* strain are used as a biocatalyst.

5. The method according to claim 4, **characterized in that** the immobilization of *E*. *coli* strain cells is carried out in a polyethyleneimine matrix covalently cross-linked with glutaraldehyde.

6. The method according to claim 5, **characterized in that** the weight ratio of polyethyleneimine and glutaraldehyde ranges from 1:1 to 3:1.

7. The method according to claim 5, **characterized in that** the weight ratio of the components of polyethyleneimine and glutaraldehyde to the *E. coli* biomass ranges from 0.25:1 to 3:1.
